# FASCICULE DE BREVET EUROPEEN

(11) **EP 0 686 407 B1**
(45) Date de publication et mention de la délivrance du brevet: **12.12.2001**
(21) Numéro de dépôt: 95440031.3
(22) Date de dépôt: 08.06.1995
(51) Int. Cl.: A61N 1/39

(54) **Appareil défibrillateur muni d'une pluralité d'ensembles connecteur/électrodes de types différents**
Defibrillator mit mehreren verschiedenartigen Stecker-/Elektroden-Anordnungen
Defibrillator with a plurality of connector/electrodes assemblies of different types

(30) Priorité: 09.06.1994 FR 9407212
(43) Date de publication de la demande: 13.12.1995
(73) Titulaire: Bruker Medical SA, 67160 Wissembourg (DE)
(72) Inventeur: Hampele, Roland, F-67500 Haguenau (FR); Kraemer, Michel, F-67360 Durrenbach (FR)
(74) Mandataire: Metz, Paul

(56) Documents cités:
- EP-A- 0 353 341
- WO-A-93/16759
- US-A- 4 837 488
- US-A- 5 181 858

## Description

La présente invention concerne le domaine des appareils de traitement des dysfonctionnements cardiaques, notamment dans le cadre de la réanimation, et a pour objet un appareil défibrillateur muni d'une pluralité d'ensembles connecteur/électrodes de types différents.

Il existe actuellement déjà un certain nombre de défibrillateurs, constitués par un boîtier contenant les différents modules fonctionnels de charge du condensateur, de contrôle et, le cas échéant, d'analyse et par différents ensembles connecteur/électrodes, comprenant chacun au moins un câble de liaison et pouvant être branchés, de manière amovible et interchangeable, sur le boîtier dudit appareil au niveau d'un site de branchement unique pourvu d'un connecteur fixe complémentaire adapté.

Parmi ces électrodes, on trouve notamment, d'une part, des électrodes à usage externe telles que les électrodes de poings ou les électrodes larges collables et, d'autre part, les électrodes à usage interne en chirurgie, en forme de cuillères, destinées à être appliquées directement sur le coeur.

Actuellement, la commande de ces appareils défibrillateurs s'effectue au moyen de boutons ou de touches de commande disposé(e)s sur le boîtier de l'appareil, sauf pour les électrodes de poings dont les boutons de déclenchement sont disposés au niveau des électrodes elles-mêmes.

En outre, un sélecteur d'énergie, permettant à l'utilisateur de déterminer le niveau de charge, en fonction du type d'électrodes et du jugement du praticien, est généralement également prévu sur le boîtier dudit appareil.

De plus, lesdits appareils défibrillateurs peuvent fonctionner soit en mode manuel (détermination du niveau d'énergie et déclenchement de la charge par action de l'utilisateur), soit en mode semi-automatique (détermination de l'énergie, déclenchement de la charge et autorisation de déclenchement de la défibrillation contrôlées par l'appareil lui-même), ce en fonction du niveau de qualification de l'utilisateur, et nécessitant de ce fait un commutateur de modes, souvent intégré au sélecteur d'énergie sous la forme d'une position de commutation supplémentaire dudit sélecteur, correspondant audit mode semi-automatique.

Or il est nécessaire de pouvoir interdire une utilisation avec commande et réglage manuel des appareils de défibrillation, notamment en milieu hospitalier, pour les personnes insuffisamment qualifiées, non habilitées ou encore habituées uniquement à une utilisation semi-automatique, par manque de pratique ou de formation.

Afin de tenter d'établir une utilisation sélective desdits appareils défibrillateurs, il a été proposé de munir ces derniers de verrous ou encore de codes d'autorisation pour accéder au mode à commande et à réglage manuels.

Toutefois, ces solutions posent des problèmes d'impossibilité de mise en oeuvre liés à l'unicité de la clé du verrou ou au nombre limité de personnes qui peuvent utiliser un appareil défibrillateur donné, ne permettant pas à une personne qualifiée, ne connaissant pas le code ou n'ayant pas la clé précitée, d'utiliser un appareil défibrillateur quelconque en mode manuel.

Par ailleurs, le mode de fonctionnement et les possibilités de réglage de l'énergie en fonction des électrodes connectées n'apparaissent pas toujours clairement et de manière évidente sur le boîtier de l'appareil défibrillateur et, la pluralité d'organes de réglage et de commande présents, ainsi que leur localisation dispersée ou intégrée sur le boîtier de l'appareil, peuvent entraîner des hésitations et des pertes de temps néfastes.

WO-A-93 16759 décrit un appareil défibrillateur muni d'une pluralité d'ensembles connecteur/électrodes de types différents, comprenant chacun au moins un câble de liaison et pouvant être branchés, de manière amovible et interchangeable, sur le boîtier dudit appareil au niveau d'un site de branchement unique pourvu d'un connecteur fixe complémentaire adapté, dans lequel une partie des organes de commande dudit appareil défibrillateur est montée sur le connecteur d'un ensemble connecteur/électrodes.

La présente invention a notamment pour but de pallier l'ensemble des inconvénients précités.

A cet effet elle a pour objet un appareil défibrillateur muni d'une pluralité d'ensembles connecteur/électrodes de types différents, comprenant chacun au moins un câble de liaison et pouvant être branchés, de manière amovible et interchangeable, sur le boîtier dudit appareil au niveau d'un site de branchement unique pourvu d'un connecteur fixe complémentaire adapté, caractérisé en ce que tous les organes de commande et, le cas échéant, de réglage du fonctionnement dudit appareil défibrillateur sont montés sur les ensembles connecteur/électrodes dont chaque type est destiné à un type d'utilisateurs autorisés et/ou à une application spécifique(s), le boîtier de l'appareil défibrillateur ne présentant que des organes de signalisation et/ou d'affichage, le cas échéant, et aucun organe de commande ou de réglage.

L'invention sera mieux comprise grâce à la description ci-après, qui se rapporte à des modes de réalisation préférés, donnés à titre d'exemples non limitatifs, et expliqués avec référence aux dessins schématiques annexés, dans lesquels:
la figure 1 est une vue de dessus d'un appareil défibrillateur conforme à l'invention;
la figure 2 est une vue schématique en élévation latérale d'un ensemble connecteur/électrodes selon l'invention;
les figures 3A et 3B sont des vues en élévation frontale de connecteurs conformes à l'invention et pouvant être assemblés, respectivement, avec des électrodes internes et avec des électrodes collables;
la figure 4 est un schéma électrique des connecteurs représentés aux figures 3A et 3B;
la figure 5 est une élévation frontale d'un connecteur selon l'invention pour un fonctionnement en semi-automatique de l'appareil défibrillateur, pouvant être assemblé avec des électrodes collables;
la figure 6 est un schéma électrique du connecteur représenté à la figure 5;
la figure 7 est une vue en élévation frontale d'un ensemble connecteur/électrodes selon l'invention, comprenant des électrodes de poings, et,
la figure 8 est un schéma électrique de l'ensemble connecteur/électrodes représenté à la figure 7.

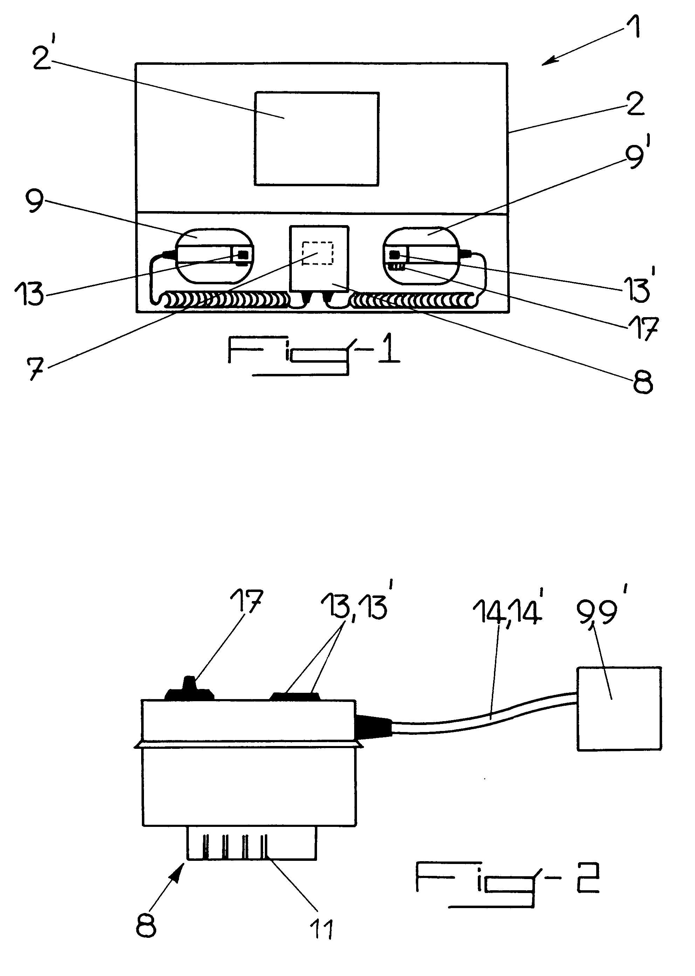

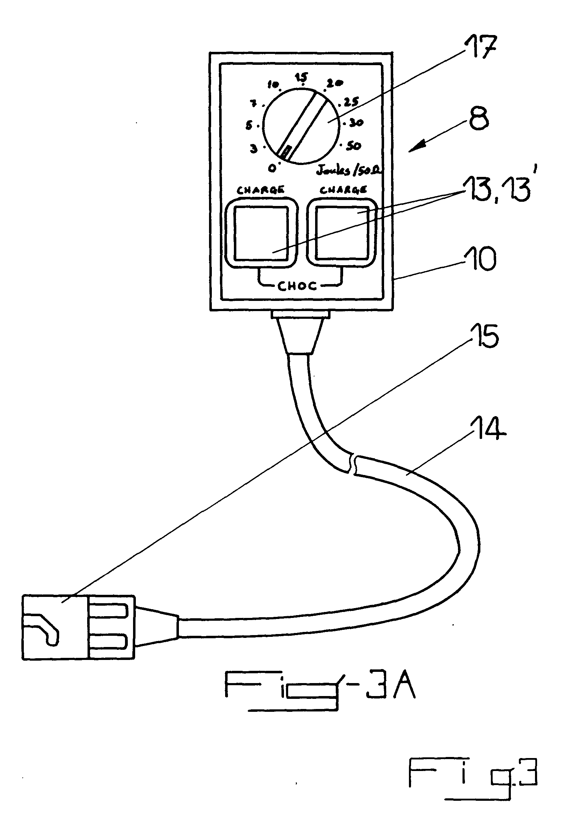

Conformément à l'invention, et comme le montrent les figures 1 à 7 des dessins annexés, l'appareil défibrillateur 1 est réalisé de telle manière que tous les organes de commande 13, 13', 18, 21 et, le cas échéant, de réglage 17 du fonctionnement dudit appareil défibrillateur 1 sont montés sur les ensembles connecteur 8/électrodes 9, 9' dont chaque type est destiné à un type d'utilisateurs autorisés et/ou à une application spécifique(s), le boîtier 2 de l'appareil défibrillateur 1 ne présentant que des organes 2' de signalisation et/ou d'affichage, le cas échéant, et aucun organe de commande ou de réglage.

Chaque type d'ensembles connecteur 8/électrodes 9, 9' étant destiné à une application et à un traitement bien définis, il est avantageux que l'appareil défibrillateur 1 présente autant de modes de fonctionnement différents, à réglage et/ou commande semi-automatique ou manuel, qu'il y a de types différents d'ensembles connecteur 8/électrodes 9, 9', le mode de fonctionnement approprié étant sélectionné automatiquement dès le branchement du connecteur 8 de l'ensemble connecteur 8/électrodes 9, 9' considéré sur le connecteur fixe 7 du boîtier 2 de l'appareil défibrillateur 1.

Ainsi, dès la mise en place d'un ensemble connecteur 8/électrodes 9, 9' par enfichage au niveau du connecteur fixe 7 (dont le site ou la région du boîtier 2 alentours peut présenter une configuration indexée complémentaire de celle du boîtier 10 du connecteur 8 de manière à faciliter un assemblage rapide et correct desdits connecteurs 7 et 8), l'unité informatique contrôlant l'appareil défibrillateur 1 peut sélectionner et suivre le protocole opératoire préprogrammé adapté au type de l'ensemble connecteur 8/électrodes 9, 9' branché, et donc à l'application envisagée, et au type d'utilisateur manipulant ledit appareil défibrillateur 1.

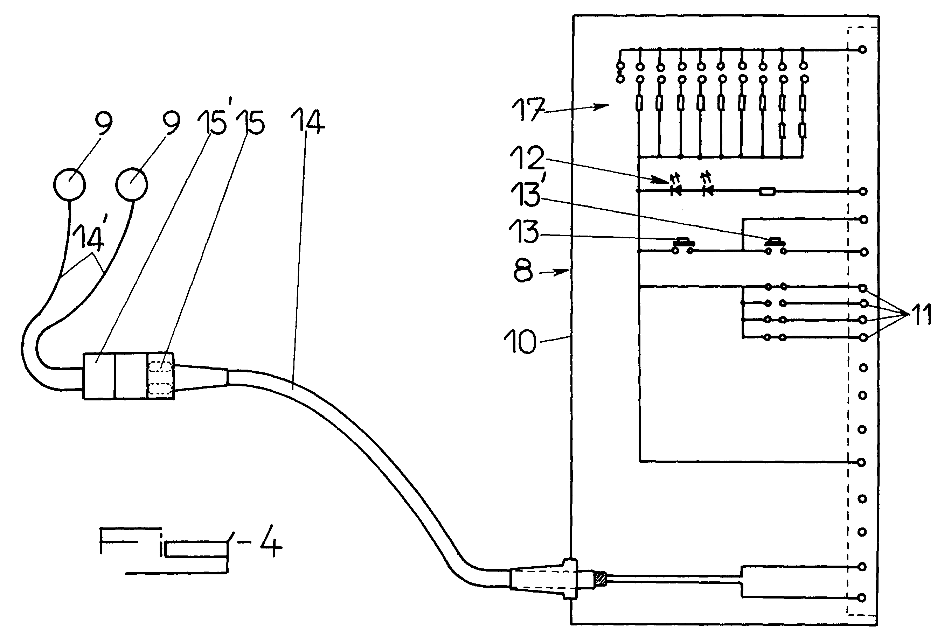

Comme le montrent les figures 4, 6 et 8 des dessins annexés, chaque connecteur 8 comporte un moyen pour sa reconnaissance par le boîtier 2 de l'appareil défibrillateur 1 lors de son branchement, spécifique au type de l'ensemble connecteur 8/électrodes 9, 9' dont il fait partie, ledit moyen de reconnaissance consistant en une définition de l'état d'au moins un point de connexion 11 dudit connecteur 8, avantageusement pendant la durée de branchement de ce dernier sur le boîtier 2 de l'appareil défibrillateur 1.

Selon une caractéristique de l'invention, représentée en partie aux figures 4, 6 et 8 des dessins annexés, le moyen de reconnaissance consiste avantageusement en un codage logique par fixation des états de points de connexion spécifiques 11 du connecteur 8, chaque code binaire ainsi obtenu identifiant de manière unique chaque type d'ensemble connecteur 8/électrodes 9, 9' et indiquant automatiquement à l'appareil défibrillateur 1, d'une part, l'occurence du branchement d'un ensemble connecteur 8/électrodes 9, 9' autorisé et, d'autre part, le type d'ensemble branché et le mode de fonctionnement correspondant.

Ainsi, la présence de quatre points de connexion 11 permet de définir, par la mise à la masse de trois point de connexion 11 et la mise à l'air du quatrième point de connexion 11, individuellement chacun des quatre types d'ensemble à brancher par la génération des codes 1000, 0100, 0010, et 0001.

En outre, le code 1111 pourra être interprété comme indiquant l'absence de branchement de tout ensemble connecteur/électrodes, et entraîner la mise en veille de l'appareil défibrillateur 1, tout code autre que ceux mentionnés ci-dessus correspondra à un état anormal, à un défaut de branchement ou à un dysfonctionnement quelconque et pourra, dès son identification, résulter en la délivrance d'un signal d'alarme.

Comme le montrent également les figures 4, 6 et 8 des dessins annexés, chaque ensemble connecteur 8/électrodes 9, 9' comporte au moins un voyant 12 de signalisation de fin de phase de charge et d'autorisation de décharge et deux boutons 13, 13' ou touches de commande couplé(e)s entre eux (elles), par exemple par montage en série, permettant de déclencher la décharge de défibrillation par appui simultané sur ces derniers.

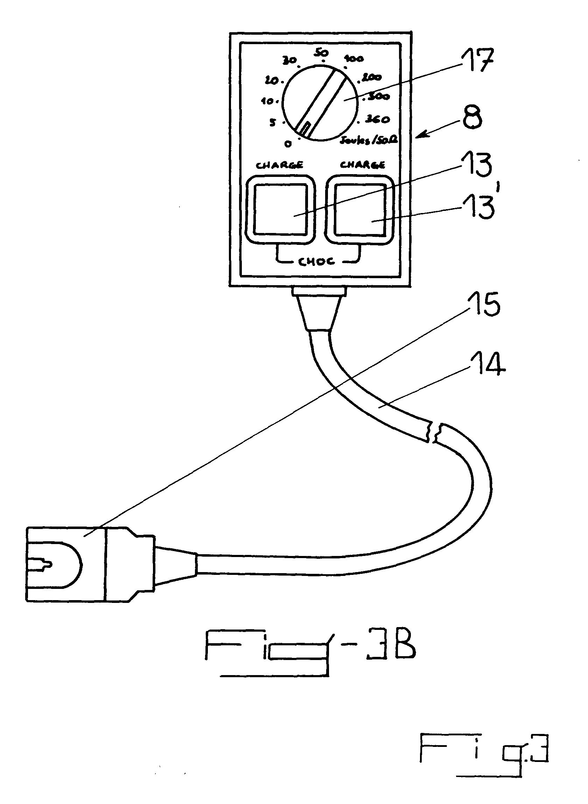

Selon une première variante de réalisation de l'invention, représentée aux figures 3A, 3B et 4 des dessins annexés, les ensembles connecteur 8/électrodes 9, 9' comprennent, dans le cadre de mise en oeuvre d'électrodes 9, 9' du type interne ou collable, un câble de liaison en deux portions 14 et 14', pouvant être assemblées entre elles, de manière amovible, par un dispositif de raccordement constitué par un couple connecteur mâle 15/connecteur femelle 15' spécifique au type d'électrodes 9, 9' à relier au connecteur 8, le boîtier 10 renfermant ce dernier comportant au moins un voyant 12 d'autorisation de décharge et deux boutons 13 et 13' ou touches couplé(e)s, par exemple par montage en série, commandant la décharge de défibrillation.

Cette disposition permet de séparer la partie de l'ensemble comprenant les électrodes 9, 9' de celle comprenant le connecteur 8, autorisant ainsi un traitement à des fins de stérilisation ou d'usage unique desdites électrodes 9, 9' destinées à être en contact avec le corps du patient.

Lorsqu'une personne, qualifiée et habilitée à effectuer une défibrillation avec commande et réglage manuels, voudra effectuer une défibrillation à l'aide d'électrodes 9, 9' internes ou collables, elle pourra mettre en oeuvre un ensemble connecteur 8/électrodes 9, 9' du type mentionné ci-dessus et dont le boîtier 10 du connecteur 8 comporte un sélecteur d'énergie 17, à variation par crans et à blocage, le déclenchement de la charge de l'appareil défibrillateur 1 étant commandé par pression sur l'un(e) des deux boutons 13, 13' ou touches couplé(e)s entre eux (elles) (Voir figures 3A, 3B et 4).

La structure et la constitution électrique des connecteurs 8 utilisés en association avec des électrodes 9, 9' internes ou collables sont identiques, comme le montrent une analyse de la figure 4 et une comparaison des figures 3A et 3B entre elles, la seule différence résidant dans l'étagement des énergies du niveau de charge, ainsi que dans les valeurs maximales disponibles.

En effet, pour un connecteur 8 destiné à être accouplé avec des électrodes 9, 9' internes, l'énergie maximale autorisée (généralement 50 Joules) et donc sélectionnable, sera nettement inférieure à celle autorisée à être sélectionnée et délivrée par l'intermédiaire d'un connecteur 8 associé à des électrodes 9, 9' larges collables destinées à être posées sur la peau du patient (généralement 360 Joules).

La prévision de connecteurs 15, 15' spécifiques, d'une part, aux ensembles connecteur 8/électrodes 9, 9' internes et, d'autre part, aux ensembles connecteur 8/électrodes 9, 9' collables, évite toute inversion au moment de l'association du connecteur 8 avec les électrodes 9, 9' du type correspondant.

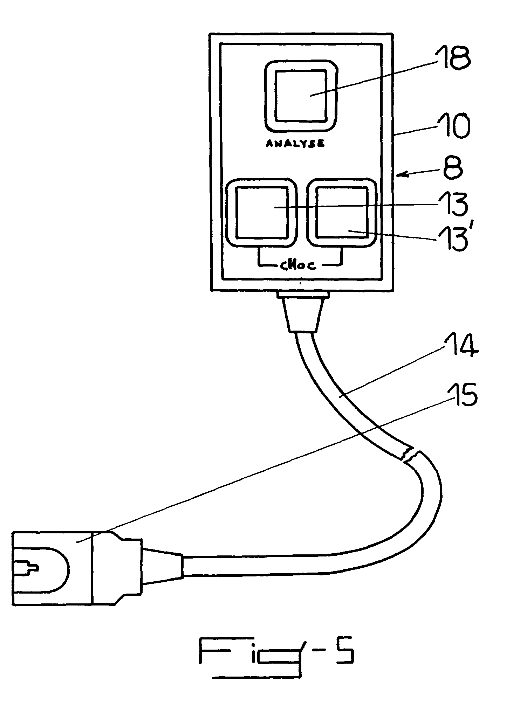

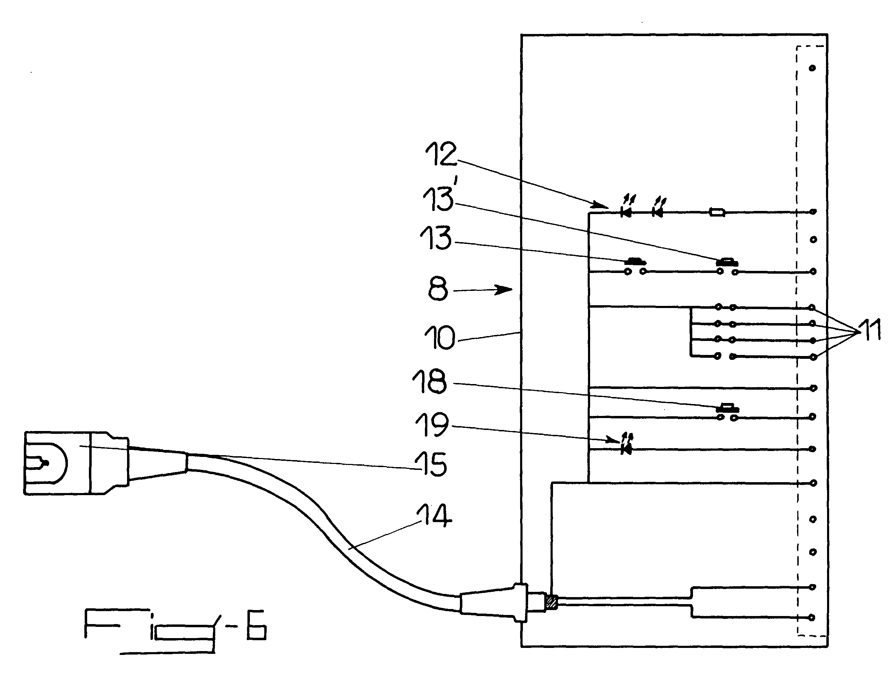

En vue de la réalisation d'opérations de défibrillation, notamment par l'intermédiaire d'électrodes 9, 9' collables, par des personnes non qualifiées ou ne possédant par la formation ou la pratique nécessaire à une exécution entièrement manuelle, il peut être prévu, comme le montrent notamment les figures 5 et 6 des dessins annexés, des ensembles connecteur 8/électrodes 9, 9' constituées par deux parties assemblées de manière amovible comme indiqué précédemment (connecteurs 15, 15' spécifiques - câble de liaison en deux parties 14 et 14'), mais dont le boîtier 10 du connecteur 8 est dépourvu de sélecteur d'énergie 17 et comporte, en remplacement, un bouton ou une touche 18 de déclenchement automatique d'un relevé d'électrocardiogramme, par l'intermédiaire des électrodes 9, 9' de défibrillation préalablement fixées sur le patient, associé(e) à un voyant de signalisation 19 de phase de mesure et d'analyse.

Ledit électrocardiogramme est analysé par ledit appareil défibrillateur 1 qui, en fonction des résultats, autorise ou non la réalisation d'une défibrillation, en déclenchant, le cas échéant automatiquement, l'opération de charge, et fixe, en association éventuellement avec des données supplémentaires (poids du patient, âge du patient ou protocoles par des normes ou recommandations, etc.) fournies audit appareil 1, la valeur de la charge ou les valeurs des charges successives.

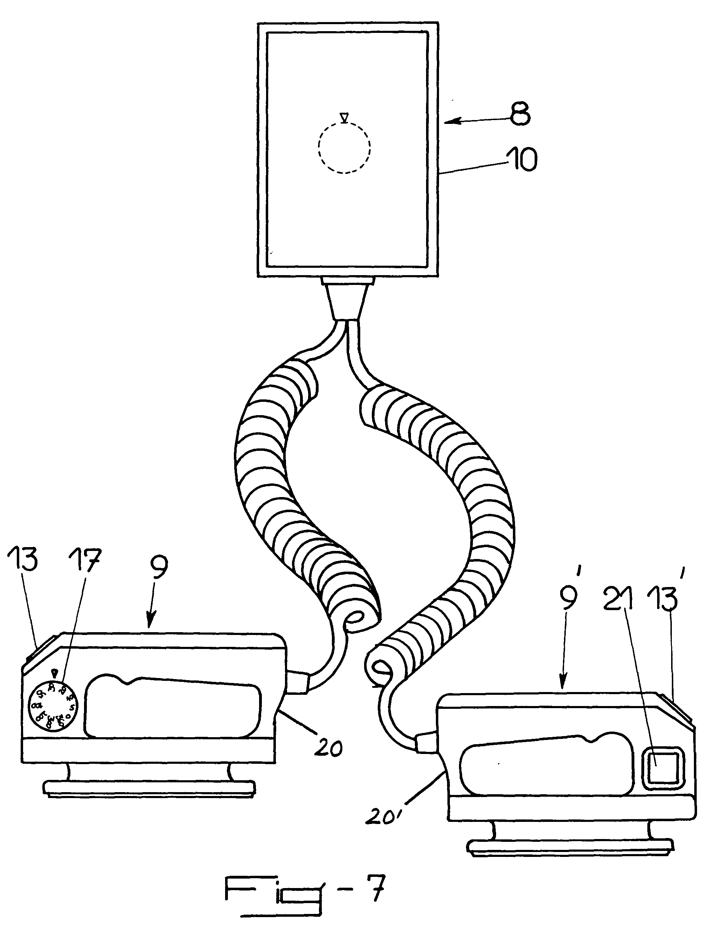

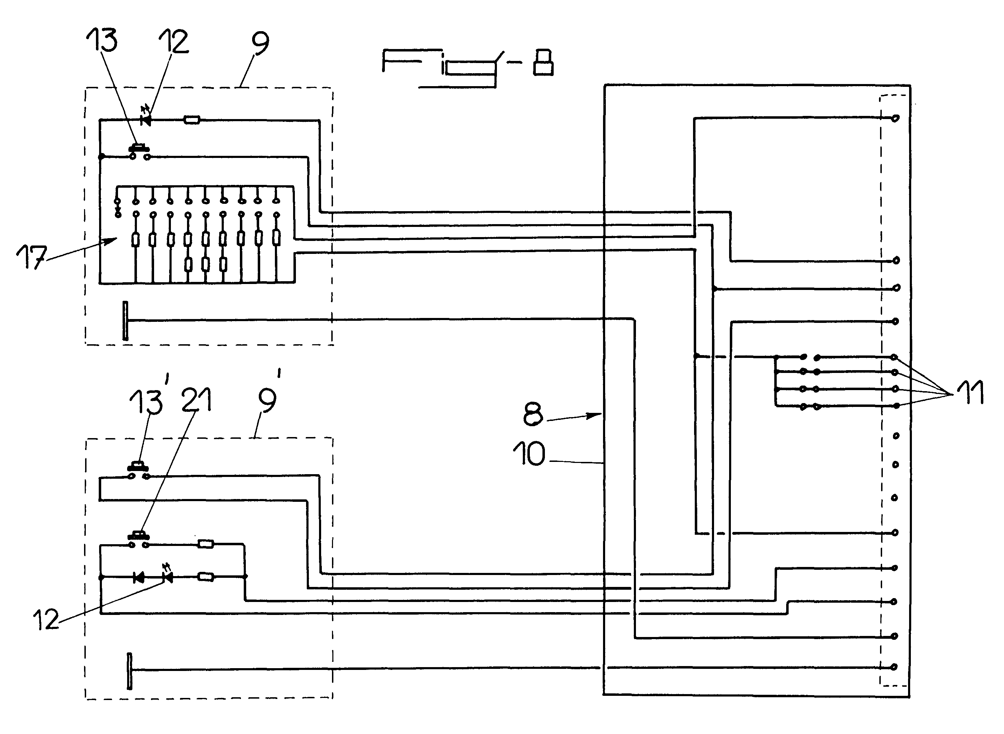

Conformément à une seconde variante de réalisation de l'invention, représentée aux figures 7 et 8 des dessins annexés et décrite dans le cadre d'ensembles connecteur 8/électrodes 9, 9' intégrant des électrodes de poing, il peut être prévu que les boîtiers de ces derniers comportent en plus d'un voyant d'indication d'autorisation de décharge 12 et de deux touches ou boutons 13, 13' à action séparée pour la commande du chargement et couplé(e)s pour la commande du déchargement, un sélecteur d'énergie 17, à variation par crans et à blocage, et une touche ou un bouton 21 de déclenchement d'un relevé d'électrocardiogramme, montés sur l'un ou l'autre desdits boîtiers 20, 20' d'électrodes 9, 9'.

Toutefois, en variante, le sélecteur d'énergie peu également être monté sur le boîtier de connecteur 8 (voir traits interrompus sur la figure 7).

Grâce à l'invention, il est donc possible d'effectuer une ségrégation entre personnes qualifiées pour des défibrillations en mode manuel et personnes autorisées uniquement à réaliser des défibrillations en mode semi-automatique, par attribution d'un ou de plusieurs ensembles connecteur 8/électrodes 9, 9' à chaque personne susceptible d'effectuer une défibrillation. En outre, chaque ensemble connecteur/électrodes est clairement identifiable de par sa constitution et les organes de commande et/ou de réglage dont il est pourvu, cette identification pouvant éventuellement être encore facilitée par le choix d'un coloris spécifique pour chaque ensemble. De plus, la localisation des organes de commande et/ou réglage sur les ensembles connecteur/électrodes permet à l'utilisateur d'évoluer en permanence dans un contexte de commande et de réglage identique et de pouvoir utiliser tout appareil défibrillateur, même éventuellement de types différents, présentant le connecteur fixe correspondant et le mode de fonctionnement adapté, ce quel que soit le lieu d'implantation dudit appareil.

Bien entendu, l'invention n'est pas limitée aux modes de réalisation décrits et représentés aux dessins annexés. Des modifications restent possibles, notamment du point de vue de la constitution des divers éléments, ou par substitution d'équivalents techniques, sans sortir pour autant du domaine de protection de l'invention tel que défini dans les revendications suivantes.

## Revendications

1. Appareil défibrillateur (1) muni d'une pluralité d'ensembles connecteur (8) électrodes (9, 9') de types différents, comprenant chacun au moins un câble de liaison et pouvant être branchés, de manière amovible et interchangeable, sur le boîtier (2) dudit appareil au niveau d'un site de branchement unique pourvu d'un connecteur fixe complémentaire adapté, tous les organes de commande (13, 13', 18, 21) et, le cas échéant, de réglage (17) du fonctionnement dudit appareil défibrillateur (1) étant montés sur les ensembles connecteur (8)/électrodes (9, 9') dont chaque type est destiné à un type d'utilisateurs autorisés et/ou à une application spécifique(s), le boîtier (2) de l'appareil défibrillateur (1) ne présentant que des organes (2') de signalisation et/ou d'affichage, le cas échéant, et aucun organe de commande ou de réglage.

2. Appareil défibrillateur selon la revendication 1, **caractérisé en ce qu'**il présente autant de modes de fonctionnement différents, à réglage et/ou commande semi-automatique ou manuel, qu'il y a de types différents d'ensembles connecteur (8)/électrodes (9, 9'), le mode de fonctionnement approprié étant sélectionné automatiquement dès le branchement du connecteur (8) de l'ensemble connecteur (8)/électrodes (9, 9') considéré sur le connecteur fixe (7) du boîtier (2) de l'appareil défibrillateur (1).

3. Appareil défibrillateur selon l'une quelconque des revendications 1 et 2, **caractérisé en ce que** chaque connecteur (8) comporte un moyen pour sa reconnaissance par le boîtier (2) de l'appareil défibrillateur (1) lors de son branchement, spécifique au type de l'ensemble connecteur (8)/électrodes (9, 9') dont il fait partie, ledit moyen de reconnaissance consistant en une définition de l'état d'au moins un point de connexion (11) dudit connecteur (8), avantageusement pendant la durée de branchement de ce dernier sur le boîtier (2) de l'appareil défibrillateur (1).

4. Appareil défibrillateur selon la revendication 3, **caractérisé en ce que** le moyen de reconnaissance consiste en un codage logique par fixation des états de points de connexion spécifiques (11) du connecteur (8), chaque code binaire ainsi obtenu identifiant de manière unique chaque type d'ensemble connecteur (8)/électrodes (9, 9') et indiquant automatiquement à l'appareil défibrillateur (1), d'une part, l'occurence du branchement d'un ensemble connecteur (8)/électrodes (9, 9') autorisé et, d'autre part, le type d'ensemble branche et le mode de fonctionnement correspondant.

5. Appareil défibrillateur selon l'une quelconque des revendications 1 à 4, **caractérisé en ce que** chaque ensemble connecteur (8)/électrodes (9, 9') comporte au moins un voyant (12) de signalisation de fin de phase de chargement et d'autorisation de décharge et deux boutons (13, 13') ou touches de commande couplé(e)s entre eux (elles), par exemple par montage en série, permettant de déclencher la décharge de défibrillation par appui simultané sur ces derniers.

6. Appareil défibrillateur selon l'une quelconque des revendications 1 à 5, **caractérisé en ce que**, dans le cadre de mises en oeuvre d'électrodes internes ou collables, les ensembles connecteur (8)/électrodes (9, 9') comprennent un câble de liaison en deux portions (14 et 14'), pouvant être assemblées entre elles, de manière amovible, par un dispositif de raccordement constitué par un couple connecteur mâle (15)/connecteur femelle (15') spécifique au type d'électrodes (9, 9') à relier au connecteur (8), le boîtier (10) du connecteur (8) renfermant ce dernier comportant au moins un voyant (12) d'autorisation de décharge et deux boutons (13 et 13') ou touches couplé(e)s, par exemple par montage en série, commandant la décharge de défibrillation.

7. Appareil défibrillateur selon la revendication 6, **caractérisé en ce que** le boîtier (10) du connecteur (8) comporte un sélecteur d'énergie (17), à variation par crans et à blocage, le déclenchement de la charge de l'appareil défibrillateur (1) étant commandé par pression sur l'un(e) des deux boutons (13, 13') ou touches couplé(e)s entre eux (elles).

8. Appareil défibrillateur selon la revendication 6, **caractérisé en ce que** le boîtier (10) du connecteur (8) comporte un bouton (18) ou une touche de déclenchement automatique d'un relevé d'électrocardiogramme, par l'intermédiaire des électrodes (9, 9') de défibrillation appropriées à être préalablement fixées sur le patient, associé(e) à un voyant de signalisation (19) de phase de mesure et d'analyse, ledit électrocardiogramme étant analysé par ledit appareil défibrillateur (1) qui, en fonction des résultats, autorise ou non la réalisation d'une défibrillation, en déclenchant, le cas échéant, automatiquement, l'opération de chargement, et fixe, en association éventuellement avec des données supplémentaires fournies audit appareil (1), la valeur de la charge ou les valeurs des charges successives.

9. Appareil défibrillateur selon l'une quelconque des revendications 1 à 5, **caractérisé en ce que**, pour des ensembles connecteur (8)/électrodes (9, 9') intégrant des électrodes de poing, les boîtiers (20, 20') de ces dernières comportent, en plus d'un voyant d'indication d'autorisation de décharge (12) et de deux touches ou boutons (13, 13') à action séparée pour la commande du chargement et couplé(e)s pour la commande du déchargement, un sélecteur d'énergie (17), à variation par crans et à blocage, et une touche ou un bouton (21) de déclenchement d'un relevé d'électrocardiogramme, montés sur l'un ou l'autre desdits boîtiers (20, 20') d'électrodes (9, 9').

## Patentansprüche

1. Defibrillator-Gerät (1) mit mehreren verschiedenartigen Elektroden (9, 9')-/Anschlußanordnungen, die jeweils mindestens ein Verbindungskabel aufweisen und an einer einzigen Anschlußeinrichtung mit einer komplementär ausgestatteten festen Anschlußstelle (7) am Gehäuse (2) des Gerätes steckbar sowie austauschbar anschließbar sind, wobei alle Bedienelemente (13, 13', 18, 21) und ggf. Steuereinrichtungen (17) des Gerätes (1) baulich mit den Elektroden (9, 9')/-Anschlußanordnungen verbunden sind, von denen jede für eine autorisierte Benutzergruppe und/oder eine bestimmte Anwendung eingerichtet ist, und das Gehäuse (2) des Defibrillator-Geräts (1) nach Bedarf nur Warn- und/oder Anzeigeeinrichtungen (2'), jedoch keine Bedienelemente oder Steuereinrichtungen aufweist.

2. Defibrillator-Gerät nach Anspruch 1, **dadurch gekennzeichnet, daß** es ebensoviel verschiedene halbautomatische oder mit manuell zu betätigenden Steuer- und/oder Bedienfunktionen ausgestattete Betriebsmodi besitzt, wie verschiedenartige Elektroden (9, 9')-/Anschlußanordnungen vorhanden sind, und daß der richtige Betriebsmodus beim Anschließen der Anschlußanordnung (8) der gewählten Elektroden (9, 9')-/Anschlußanordnung an der festen Anschlußstelle (7) am Gehäuse (2) des Defibrillator-Gerätes (1) automatisch bestimmt wird.

3. Defibrillator-Gerät nach einem der Ansprüche 1 und 2, **dadurch gekennzeichnet, daß** jede Anschlußanordnung (8) ein durch das Gehäuse (2) des Defibrillator-Gerätes (1) im Moment des Anschließens erkennbares, für die entsprechende Elektroden (9, 9')-/Anschlußanordnung spezifisches und ein Teil derselben bildendes Erkennungsmerkmal aufweist, und daß das Erkennungsmerkmal aus einer Zustandsdefinition wenigstens einer Anschlußstelle (11) der Anschlußanordnung (8) vorteilhafterweise während deren Anschlußdauer an dem Gehäuse (2) des Defibrillator-Gerätes (1) besteht.

4. Defibrillator-Gerät nach Anspruch 3, **dadurch gekennzeichnet, daß** das Erkennungsmerkmal aus einer logischen Kodierung durch Festlegung der Zustände spezifischer Anschlußpunkte (11) der Anschlußanordnung (8) besteht, wobei jeder auf diese Weise gewonnene Binärcode die entsprechende Elektroden (9, 9')-/Anschlußanordnung eindeutig identifiziert und einerseits am Defibrillator-Gerät (1) die Anschlußlage einer zulässigen Elektroden (9, 9')-/Anschlußanordnung, andererseits den Typ der angeschlossenen Anordnung und den zugehörigen Betriebsmodus automatisch anzeigt.

5. Defibrillator-Gerät nach einem der Ansprüche 1 bis 4, **dadurch gekennzeichnet, daß** jede Elektroden (9, 9')-/Anschlußanordnung mindestens eine Kontrollampe zum Anzeigen des Endes der Aufladephase und zur Freigabe der Entladung sowie zwei miteinander gekuppelte, z.B. in Reihe geschaltete Bedienknöpfe (13, 13') oder Bedientasten zum Auslösen der Defibrillationsentladung durch gleichzeitiges Drücken beider Knöpfe aufweist.

6. Defibrillator-Gerät nach einem der Ansprüche 1 bis 5, **dadurch gekennzeichnet, daß** im Rahmen des Einsatzes interner oder klebbarer Elektroden die Elektroden (9, 9')-/Anschlußanordnungen ein zweiteiliges Verbindungskabel (14, 14') aufweisen, dessen Teile mittels einer für den mit der Anschlußanordnung (8) zu verbindenden Typ von Elektroden (9, 9') spezifischen Kupplung aus korrelierenden Kupplungsteilen (15, 15') verbindbar sind, und daß das die Anschlußanordnung (8) einschließende Gehäuse (10) mindestens eine Kontrollampe (12) zur Freigabe der Entladung sowie zwei miteinander gekuppelte, z.B. in Reihe geschaltete Bedienknöpfe (13, 13') oder Bedientasten zum Auslösen der Defibrillationsentladung aufweist.

7. Defibrillator-Gerät nach Anspruch 6, **dadurch gekennzeichnet, daß** das Gehäuse (10) der Anschlußanordnung (8) einen stufenweise verstellbaren und blockierbaren Energieregler aufweist, und daß der Aufladevorgang des Defibrillator-Gerätes (1) durch Drücken eines bzw. einer der miteinander gekuppelten Knöpfe (13, 13') bzw. Tasten ausgelöst wird.

8. Defibrillator-Gerät nach Anspruch 6, **dadurch gekennzeichnet, daß** das Gehäuse (10) der Anschlußanordnung (8) einen Knopf (18) bzw. eine Taste zum automatischen Auslösen einer Elektrokardiogrammaufnahme mittels zuvor am Patienten anbringbarer Defibrillationselektroden zusammen mit einer Kontrollampe zur Anzeige der Meß- und Analysephase aufweist, und daß das Elektrokardiogramm von dem Defibrillator-Gerät (1) auswertbar ist, das in Abhängigkeit von den Resultaten die Durchführung einer Defibrillation genehmigt oder nicht und ggf. automatisch den Aufladevorgang auslöst und die Ladungsstärke oder die Stärke aufeinanderfolgender Ladungen gegebenenfalls in Verbindung mit weiteren Eingaben an dem Gerät (1) festlegt.

9. Defibrillator-Gerät nach einem der Ansprüche 1 bis 5, **dadurch gekennzeichnet, daß** bei Elektroden (9, 9')-/Anordnungen mit integrierten Handelektroden deren Elektroden-Gehäuse (20, 20') neben einer Kontrollampe zur Anzeige der Entladebereitschaft und zwei Tasten oder Knöpfen (13, 13') zum Auslösen des Aufladevorgangs bei Einzelbetätigung oder des Entladevorgangs bei gleichzeitiger Betätigung zusätzlich einen stufenweise verstellbaren oder blockierbaren Energieregler sowie eine Taste oder einen Knopf (21) zur Auslösung einer Elektrokardiogrammaufnahme aufweist, die an einem der beiden Gehäuse (20, 20') der Elektroden (9, 9') angeordnet sind.

## Claims

1. Defibrillator (1) having a plurality of connector (8)/electrodes (9, 9') assemblies of different types, each of which has at least one connecting cable and can be connected, in a removable and interchangeable manner, to the case (2) of said defibrillator at a single connection site provided with a suitable complementary fixed connector, all the devices for controlling (13, 13', 18, 21) and, where appropriate, for regulating (17) the operation of said defibrillator (1) being mounted on the connector (8)/ electrodes (9, 9') assemblies, each type of which is intended for a specific authorised type of user and/or for a specific application, the case (2) of the defibrillator (1) having only members (2') for indicating and/or displaying, where appropriate, and no control or regulating members.

2. Defibrillator according to claim 1, **characterised in that** it has the same number of different operating modes, which are regulated and/or controlled semi-automatically or manually, as there are different types of connector (8)/electrodes (9, 9') assemblies, the appropriate operating mode being selected automatically as soon as the connector (8) of the connector (8)/electrodes (9, 9') assembly in question is connected to the fixed connector (7) of the case (2) of the defibrillator (1).

3. Defibrillator according to either claim 1 or claim 2, **characterised in that** each connector (8) has a means by which it can be recognised by the case (2) of the defibrillator (1) when it is connected, which means is specific to the type of connector (8)/electrodes (9, 9') assembly of which it is part, the said recognition means consisting in a definition of the state of at least one connecting point (11) of the said connector (8), advantageously throughout the period of connection of the latter to the case (2) of the defibrillator (1).

4. Defibrillator according to claim 3, **characterised in that** the recognition means consists in a logical code determined by the states of specific connecting points (11) of the connector (8), each binary code so obtained identifying each type of connector (8)/electrodes (9, 9') assembly in a unique manner and automatically indicating to the defibrillator (1) on the one hand that an authorised connector (8)/electrodes (9, 9') assembly has been connected and on the other hand the type of assembly connected and the corresponding mode of operation.

5. Defibrillator according to any one of claims 1 to 4, **characterised in that** each connector (8)/electrodes (9, 9') assembly has at least one indicator (12) for indicating the end of a charging phase and authorising discharge, and two control buttons (13, 13') or keys which are coupled together, for example by being mounted in series, and which allow a defibrillation discharge to be started by simultaneous depression thereof.

6. Defibrillator according to any one of claims 1 to 5, **characterised in that**, in the context of the use of internal or adhesive electrodes, the connector (8)/electrodes (9, 9') assemblies have a connecting cable in two portions (14 and 14') which can be joined together, in a removable manner, by a joining device formed by a male connector (15)/female connector (15') pair specific to the type of electrodes (9, 9') to be connected to the connector (8), the case (10) of the connector (8) containing the latter having at least one indicator (12) for authorising discharge and two buttons (13 and 13') or keys which are coupled, for example by being mounted in series, and which control the defibrillation discharge.

7. Defibrillator according to claim 6, **characterised in that** the case (10) of the connector (8) has a power selector (17), with variation by notches and with locking, the start of charging of the defibrillator (1) being controlled by pressure on one of the two buttons (13, 13') or keys which are coupled together.

8. Defibrillator according to claim 6, **characterised in that** the case (10) of the connector (8) has a button (18) or a key for automatically triggering an electrocardiogram reading, by way of the defibrillation electrodes (9, 9') which are suitable for attachment to the patient beforehand, which button (18) or key is associated with an indicator (19) for indicating a measuring and analysis phase, the said electrocardiogram being analysed by said defibrillator (1) which, as a function of the results, authorises, or does not authorise, defibrillation by, where appropriate, automatically starting the charging operation, and determines, optionally in association with additional data supplied to said defibrillator (1), the value of the charge or the values of successive charges.

9. Defibrillator according to any one of claims 1 to 5, **characterised in that**, for connector (8)/electrodes (9, 9') assemblies incorporating chest electrodes, the cases (20, 20') thereof have, in addition to a discharge authorisation indicator (12) and two keys or buttons (13, 13') which act separately for controlling charging and are coupled for controlling discharge, a power selector (17), with variation by notches and with locking, and a key or a button (21) for triggering an electrocardiogram reading, mounted on one or other of said cases (20, 20') of the electrodes (9, 9').
